(19) Europäisches Patentamt
European Patent Office
Office européen des brevets

(11) Veröffentlichungsnummer: **0 130 149**
**B1**

(12) **EUROPÄISCHE PATENTSCHRIFT**

(45) Veröffentlichungstag der Patentschrift: **12.04.89**

(51) Int. Cl.⁴: **C 07 D 207/34**, A 01 N 43/36

(21) Anmeldenummer: **84810289.3**

(22) Anmeldetag: **12.06.84**

(54) **3-Phenyl-4-cyanopyrrol-Derivate, Verfahren zu deren Herstellung und deren Verwendung als Mikrobizide.**

(30) Priorität: **17.06.83 CH 3331/83**

(43) Veröffentlichungstag der Anmeldung: **02.01.85 Patentblatt 85/1**

(45) Bekanntmachung des Hinweises auf die Patenterteilung: **12.04.89 Patentblatt 89/15**

(84) Benannte Vertragsstaaten: **BE CH DE FR IT LI LU NL SE**

(56) Entgegenhaltungen:
**EP-A- 0 019 978**
**EP-A- 0 092 890**
**DE-A- 2 927 480**
**GB-A- 2 013 187**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Nyfeler, Robert, Dr., Bärenfelserstrasse 8, CH-4057 Basel (CH)**

EP 0 130 149 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N-alkylierte 3-Phenyl-4-cyanopyrrol-derivate der nachstehenden Formel I, deren Herstellung, sowie mikrobizide Mittel, die als Wirkstoff mindestens eine der Titelverbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Es werden hierin neue Verbindungen der allgemeinen Formel I

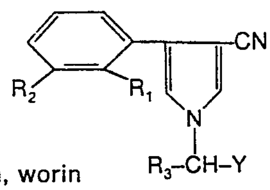

(I)

beschrieben, worin

$R_1$ und $R_2$ unabhängig voneinander Wasserstoff, Halogen, Methoxy oder Methylthio stehen;

$R_3$ Wasserstoff, oder $C_1$–$C_4$-Haloalkyl bedeutet;

Y für Hydroxy, Halogen oder die Gruppe –O–C(O)–$R_4$ steht und

$R_4$ H, $C_1$–$C_4$-Alkyl, $C_1$–$C_3$-Haloalkyl, $C_1$–$C_2$-Alkoxycarbonyl oder die Gruppe –CH($R_5$)–X$R_6$ bedeutet; wobei

X für Sauerstoff oder Schwefel steht;

$R_5$ Wasserstoff oder Methyl bedeutet; und

$R_6$ für $C_1$–$C_4$-Alkyl, ($C_1$–$C_2$-Alkoxy)-$C_1$–$C_2$-alkyl, $C_3$–$C_4$-Alkenyl, oder Phenyl steht.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl usw., sowie ihre Isomeren, wie z.B. Isopropyl, Isobutyl, tert.-Butyl, Isopentyl usw. Haloalkyl steht für einen einfach bis perhalogenierten Alkylsubstituenten, wie z.B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$–$CHCl_2$, $CH_2CH_2F$, $CJ_3$ usw. Unter Halogen soll hier und im folgenden Fluor, Chlor, Brom oder Jod, vorzugsweise Fluor, Chlor oder Brom, verstanden werden. Alkenyl steht z.B. für Vinyl, Propenyl-(1), Allyl, Butenyl-(1), Butenyl-(2), Butenyl-(3), usw. sowie für Alkylketten, die durch mehrere C=C-Doppelbindungen unterbrochen sind.

Die Verbindungen der Formel I sind unter Normalbedingungen stabile Öle, Harze oder überwiegend kristalline Feststoffe, die sich durch äußerst wertvolle mikrobizide Eigenschaften auszeichnen. Sie lassen sich vor allem unter Freilandbedingungen auf dem Agrarsektor oder verwandten Gebieten kurativ und insbesondere präventiv zur Bekämpfung pflanzenschädigender Mikroorganismen einsetzen. Die erfindungsgemäßen Wirkstoffe der Formel I zeichnen sich in weiten Anwendungskonzentrationen durch eine hohe fungizide Aktivität und problemlose Anwendung im Freiland aus.

Aufgrund ihrer ausgeprägten mikrobiziden Aktivität sind folgende Substanzgruppen in steigender Reihenfolge zunehmend bevorzugt:

a) Verbindungen der Formel I, worin $R_1$ in der 2-Position und $R_2$ in der 3-Position fixiert sind und unabhängig voneinander für H, Halogen, Methoxy, oder Methylthio stehen; $R_3$ Wasserstoff, oder $C_1$–$C_4$-Haloalkyl bedeutet; und Y für Hydroxy, Halogen oder die Gruppe –O–C(O)–$R_4$ steht; wobei $R_4$ für $C_1$–$C_4$-Alkyl, $C_1$–$C_3$-Haloalkyl, $C_1$–$C_2$-Alkoxycarbonyl oder die Gruppe –CH($R_5$)–X$R_6$ bedeutet; wobei

X für Sauerstoff oder Schwefel steht;

$R_5$ Wasserstoff oder Methyl bedeutet; und

$R_6$ für $C_1$–$C_4$-Alkyl, ($C_1$–$C_2$-Alkoxy)-$C_1$–$C_2$-alkyl, $C_3$–$C_4$-Alkenyl, oder Phenyl steht.

b) Verbindungen der Formel I, worin $R_1$ für H, 2-Cl, 2-Methoxy oder 2-Methylthio steht; $R_2$ H oder 3-Cl bedeutet; $R_3$ für Wasserstoff, oder $C_1$–$C_4$-Haloalkyl steht; und Y OH, Chlor oder die Gruppe –O–C(O)–$R_4$ bedeutet, wobei $R_4$ für $C_1$–$C_4$-Alkyl, $C_1$–$C_3$-Haloalkyl, $C_1$–$C_2$-Alkoxycarbonyl oder die Gruppe –$CH_2$–O$R_6$ bedeutet; wobei $R_6$ für $C_1$–$C_4$-Alkyl, ($C_1$–$C_2$-Alkoxy)-$C_1$–$C_2$-alkyl, oder unsubstituiertes oder Phenyl steht.

c) Verbindungen der Formel I, worin $R_1$ für H, 2-Cl, 2-Methoxy oder 2-Methylthio steht; $R_2$ H oder 3-Cl bedeutet; $R_3$ für Wasserstoff, oder $CCl_3$ steht und Y OH, Chlor oder die Gruppe –O–C(O)–$R_4$ bedeutet; wobei $R_4$ für $C_1$–$C_4$-Alkyl, $C_1$–$C_2$-Haloalkyl, Methoxycarbonyl oder die Gruppe –$CH_2OR_6$ bedeutet; wobei $R_6$ für $C_1$–$C_4$-Alkyl, Methoxymethyl, Ethoxymethyl, Methoxyethyl, Allyl, oder Phenyl steht.

Besonders bevorzugte Einzelsubstanzen sind z.B.:

Nachfolgend genannte Einzelsubstanzen a bis u sind insbesondere wegen ihrer starken pflanzenfungiziden Wirkung unter Freilandbedingungen besonders bevorzugt:

a) N-(Hydroxymethyl)-3-(3-chlorphenyl)-4-cyanopyrrol,
b) N-(1-Acetyloxy-2,2,2-trichlorethyl)-3-(2,3-dichlorphenyl)-4-cyanopyrrol,
c) N-(1-Hydroxy-2,2,2-trichlorethyl)-3-(2-methylthiophenyl)-4-cyanopyrrol,
d) N-(1-Hydroxy-2,2,2-trichlorethyl)-3-(2,3-dichlorphenyl)-4-cyanopyrrol,
e) N-(1-Hydroxy-2,2,2-trichlorethyl)-3-(2-methoxyphenyl)-4-cyanopyrrol,
f) N-(1-Methoxyacetyloxy-2,2,2-trichlorethyl)-3-(2,3-dichlorphenyl)-4-cyanopyrrol,
g) N-(1-Hydroxy-2,2,2-trichlorethyl)-3-(3-methoxyphenyl)-4-cyanopyrrol,
h) N-(Methoxyacetyloxymethyl)-3-(2,3-dichlorphenyl)-4-cyanopyrrol,
i) N-(1-Hydroxy-2,2,2-trichlorethyl)-3-(2-chlorphenyl)-4-cyanopyrrol,
k) N-(Chlormethyl)-3-(3-methoxyphenyl)-4-cyanopyrrol,
l) N-[1-(n-Butylcarbonyloxy)-2,2,2-trichlorethyl]-3-(2,3-dichlorphenyl)-4-cyanopyrrol,
m) N-(1,2,2,2-Tetrachlorethyl)-3-(3-methoxyphenyl)-4-cyanopyrrol,
n) N-[1-(n-Propoxyacetyloxy)-2,2,2-trichlorethyl]-3-(2,3-dichlorphenyl)-4-cyanopyrrol,
o) N-(1,2,2,2-Tetrachlorethyl)-3-(3-bromphenyl)-4-cyanopyrrol,

p) N-[1-(n-Butoxyacetyloxy)-2,2,2-trichlorethyl]-3-(2,3-dichlorphenyl)-4-cyanopyrrol,
q) N-[1-(Isopropoxyacetyloxy)-2,2,2-trichlorethyl]-3-(2-chlorphenyl)-4-cyanopyrrol,
r) N-[1-(n-Butoxyacetyloxy)ethyl]-3-(2,3-dichlorphenyl)-4-cyanopyrrol,
s) N-[1-(Methoxyacetyloxy)-2,2,2-trichlorethyl]-3-(2-methylthiophenyl)-4-cyanopyrrol,
t) N-[1-(Propargyloxyacetyloxy)-2,2,2-trichlorethyl]-3-(2,3-dichlorphenyl)-4-cyanopyrrol,
u) N-(Methoxyacetyl)-3-(2-chlorphenyl)-4-cyanopyrrol.

Die neuen Verbindungen der Formel I werden erfindungsgemäß dadurch hergestellt, daß man ein 3-Phenyl-4-cyanopyrrol-derivat der Formel II

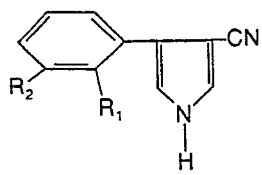
(II)

durch Reaktion mit einem Aldehyd der Formel III

$$R_3\text{–CHO} \qquad (III)$$

in ein Hydroxyderivat der Formel Ia

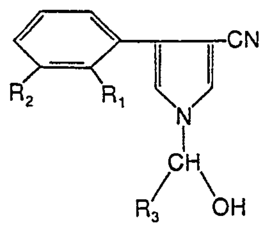
(Ia)

überführt und letzteres, sofern gewünscht, durch Austausch der freien OH-Gruppe gegen einen andern Rest Y in eines der übrigen Produkte der Formel I überführt, wobei dieser Austausch dadurch erfolgt, daß man entweder eine Verbindung der Formel Ia mit einer Säure der Formel IV

$$R_4\text{–COOH} \qquad (IV)$$

oder vorzugsweise mit einem ihrer reaktionsfähigen Säurederivate, insbesondere Säurehalogenide, wie z.B. Säurechlorid oder Säurebromid oder mit ihrem Säureanhydrid in ein Acyloxy-Produkt der Formel Ib

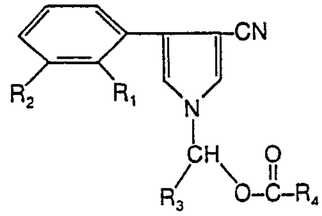
(Ib)

überführt, oder dadurch, daß man die freie OH-Gruppe in einer Verbindung der Formel Ia zuerst auf übliche Weise gegen ein Halogen austauscht, vorzugsweise Chlor oder Brom, und, sofern gewünscht, das nunmehr halogenierte Produkt durch Reaktion mit einem Salz der Formel V

$$R_4\text{–COO}^-M^+ \qquad (V)$$

in eine Verbindung der Formel Ib überführt, hierbei haben die Substituenten in den Formeln Ia, Ib, II, III, IV und V die unter Formel I angegebenen Bedeutungen, $M^+$ steht für ein Metallkation, vorzugsweise für ein Erdalkali- oder insbesondere Alkalimetallkation, wie z.B. $Ca^{++}$, $Mg^{++}$, $Na^+$ oder $K^+$.

Die Reaktion von Verbindungen der Formel II mit Aldehyden der Formel III kann in An- oder Abwesenheit reaktionsinerter Lösungsmittel oder Lösungsmittelgemische durchgeführt werden. Es eignen sich beispielsweise aromatische Kohlenwasserstoffe, wie Benzol, Toluol oder Xylole; halogenierte Kohlenwasserstoffe, wie Chlorbenzol; aliphatische Kohlenwasserstoffe wie Petrolether; Ether und etherartige Verbindungen, wie Dialkylether (Diethylether, Diisopropylether, tert.-Butylmethylether usw.), Furan, Dimethoxyethan, Dioxan, Tetrahydrofuran; Dimethylformamid usw.

Die Umsetzung von Verbindungen der Formel II mit Verbindungen der Formel III wird vorteilhafterweise ohne Lösungsmittel, jedoch mit einer überschüssigen Menge des Aldehyds III durchgeführt. Je nach Art des Aldehyds III arbeitet man in Lösung oder in der Schmelze. Hierbei wirkt sich die Zugabe saurer oder basischer Katalysatoren günstig auf die Reaktionsgeschwindigkeit aus. Als saure Katalysatoren kommen z.B. wasserfreie Halogenwasserstoffe und Mineralsäuren, wie HCl, HBr oder $H_2SO_4$ aber auch konzentrierte Salzsäure in Frage. Als basische Katalysatoren können z.B. Trialkylamine (Trimethylamin, Triethylamin, Dimethylethylamin, usw.), Alkali- und Erdalkalicarbonate (wie $Na_2CO_3$, $BaCO_3$, $MgCO_3$, $K_2CO_3$, usw.) oder Alkalialkoholate (wie $NaOCH_3$, $NaOC_2H_5$, KO(iso-$C_3H_7$), KO(t.-Butyl)) eingesetzt werden. Die Temperaturen liegen bei dieser Umsetzung im allgemeinen zwischen 0° und 200 °C, vorzugsweise 0° und 160 °C, und die Reaktionsdauer beträgt 1 bis 24 Stunden, insbesondere 1 bis 4 Stunden.

Der Austausch der freien Hydroxylgruppe in den Verbindungen der Formel Ia gegen eine Gruppe Y wird bevorzugt in einem reaktionsinerten Lösungsmittel durchgeführt. Beispiele solcher Lösungsmittel sind: Aromatische und aliphatische Kohlenwasserstoffe wie Benzol, Toluol, Xylole, Petrolether, Ligroin oder Cyclohexan; halogenierte Kohlenwasserstoffe wie Chlorbenzol, Methylenchlorid, Ethylenchlorid, Chloroform, Tetrachlorkohlenstoff oder Tetrachlorethylen; Ether und etherartige Verbindungen wie Diethylether, Diisopropylether, T-Butylmethylether, Dimethoxyethan, Dioxan, Tetrahydrofuran oder Anisol; Ester wie Ethylacetat, Propylacetat oder Butylacetat; Nitrile wie Acetonitril; oder Verbindungen wie Dimethylsulfoxid, Dimethylformamid und Gemische solcher Lösungsmittel untereinander.

Die Einführung der Gruppe Y erfolgt nach üblichen Methoden. Bedeutet Y Chlor, so wird als Reagenz z.B. Phosphoroxychlorid, Phosphortrichlorid, Phosphorpentachlorid oder vorzugsweise Thionylchlorid eingesetzt. Man arbeitet im allgemeinen bei Temperaturen von 0° bis +120 °C. Im Falle von Y = Brom verwendet man bevorzugt Phosphortribromid oder Phosphorpentabromid und führt die Reaktion bei 0° bis +50 °C durch. Steht Y für die Gruppe $-O-C(O)-R_4$, so wird als Reagenz üblicherweise das entsprechende Säurehalogenid, insbesondere Säurechlorid, eingesetzt. Hierbei ist es zweckmäßig, wenn die Reaktion bei Temperaturen von −20° bis +50 °C, vorzugsweise −10° bis +30 °C und in Gegenwart einer schwachen Base wie Pyridin oder Triethylamin durchgeführt wird. Ferner können als Katalysatoren 4-Dialkylaminopyridine wie 4-Dimethyl- oder 4-Diethylaminopyridin zur Beschleunigung der Reaktion zugesetzt werden.

Die Umsetzung von Verbindungen der Formel I, worin Y für Halogen, insbesondere Chlor oder Brom steht mit Salzen der Formel V erfolgt üblicherweise in Gegenwart eines üblichen inerten Lösungsmittels oder Lösungsmittelgemisches. Beispiele solcher Lösungsmittel sind: Aromatische und aliphatische Kohlenwasserstoffe wie Benzol, Toluol, Xylole, Petrolether, Ligroin oder Cyclohexan; Ether und etherartige Verbindungen wie Dialkylether z.B. Diethylether, Diisopropylether, T-Butylmethylether, Dimethoxyethan, Dioxan, Tetrahydrofuran oder Anisol; Ester wie Ethylacetat, Propylacetat oder Butylacetat; Nitrile wie Acetonitril; oder Verbindungen wie Dimethylsulfoxid, Dimethylformamid und Gemische solcher Lösungsmittel untereinander.

Die Zugabe katalytischer Mengen eines Kronenethers, wie z.B. 18-Krone-6 oder 15-Krone-5 wirken sich bei dieser Umsetzung günstig auf den Reaktionsverlauf aus. Die Reaktionstemperaturen liegen im allgemeinen zwischen 0° und 150 °C, vorzugsweise 20 bis 80 °C. Die Reaktionsdauer beträgt 1 bis 24 Stunden.

In einer bevorzugten Ausführungsform wird die Herstellung von Verbindungen der Formel Ib, insbesondere jener mit $R_3 = CCl_3$ oder $R_3 = H$, ausgehend von Verbindungen der Formel II, im sogenannten Eintopfverfahren durchgeführt. Hierbei arbeitet man vorteilhafterweise in einem der oben genannten inerten Lösungs- oder Verdünnungsmittel, besonders geeignet sind z.B. etherartige Verbindungen, wie Tetrahydrofuran und in Gegenwart einer schwachen Base, wie Trialkylamin (Triethylamin) oder Pyridin. Als Reagenzien werden Chloral oder Paraformaldehyd eingesetzt. Die Reaktion kann durch Zugabe eines Katalysators wie z.B. 1,8-Diazabicyclo[5.4.0]undec-7-en [=DBU] beschleunigt werden. Die Temperaturen liegen bei dieser ersten Reaktionsstufe bei −20° bis +100 °C, bevorzugt 0° bis 50 °C und die Reaktionszeit bei 0,5 bis 2 Stunden. Es entsteht intermediär ein Hydroxyderivat der Formel Ia, das nicht isoliert wird, sondern in der selben Reaktionslösung bei −30° bis +30 °C, vorzugsweise −10° bis 0 °C und in Gegenwart katalytischer Mengen eines 4-Dialkylaminopyridins, vorzugsweise 4-Dimethylaminopyridin, mit einer Verbindung der Formel IV umgesetzt wird. Die Reaktionsdauer für diese 2. Stufe liegt bei 0,5 bis 16 Stunden.

Die Ausgangssubstanzen der Formeln III, IV und V sind allgemein bekannt oder werden nach an sich bekannten Methoden hergestellt.

Ein Teil der Verbindungen der Pyrrole der Formel II ist aus der Literatur bekannt. So werden z.B. die Herstellungsmethode und die chemischen Eigenschaften von 4-Cyano-3-phenyl-pyrrol in Tetrahedron-Letters No. 52, S. 5337–5340 (1972) beschrieben. Über eine biologische Wirksamkeit der Verbindung wird nicht berichtet.

N-Acetylierte substituierte 3-Phenyl-4-cyanopyrrol-derivate sind aus der Literatur bekannt, so werden beispielsweise Pyrrole der Formel VI

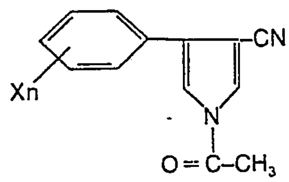

(VI),

worin X ein Halogenatom, eine niedere Alkyl- oder niedere Halogenalkylgruppe bedeutet und n für 0, 1 oder 2 steht, in der DE-OS 2 927 480 als Fungizide beschrieben.

In der EP-A-92 890 ist ein Verfahren zur Herstellung von, an der 4-Stelle gegebenenfalls halogensubstituierten 3-Phenylpyrrol-Derivaten beschrieben, welche als Fungizide oder Fungizid-Zwischenprodukte verwendet werden. Die Herstellung erfolgt durch Decarboxylierung der entsprechenden 2,5-Dicarbonsäuren.

In der EP-A-19 987 sind N-Acyl-3-phenyl-4-chlorpyrrol-Derivate als Fungizide beschrieben.

In der GB-A 2 013 187 sind fungizid wirksame 3-Phenyl-4-chlorpyrrole und deren N-Acetyl-Derivate beschrieben.

Ferner sind Pyrrol-derivate der Formel VII

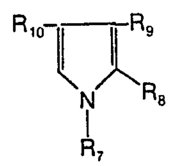

(VII)

mit

$R_7$ = Acyl, Alkoxycarbonylalkyl ...

$R_8$, $R_9$ = H, Aryl ....

$R_{10}$ = Hydroxy, Mercapto

aus der GB-PS 2 078 761 als Hitze- und Lichtstabilisatoren für PVC-Kunststoffe bekannt.

Weitere Pyrrol-derivate der Formel VIII

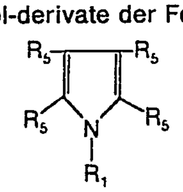

(VIII)

mit

$R_1$ = Alkyl, gegebenenfalls durch Acyloxy substituiert ...

$R_5$ = H, Alkyl, CN ...

werden als Polymerisationskatalysatoren für Vinylchlorid in der DE-OS 2 028 363 genannt.

Die bekannen Pyrrol-Derivate sind entweder gegenüber Pflanzenpathogenen unwirksam oder zeigen zwar im Gewächshaus eine deutliche pflanzenfungizide Wirkung, die jedoch unter Freilandbedingungen auf Grund ihrer Instabilität gegenüber Umwelteinflüssen nicht reproduziert ist. Sie sind daher für den praktischen Einsatz in der Landwirtschaft im Gartenbau oder in verwandten Einsatzgebieten nicht geeignet. Die erfindungsgemäßen, neuen Pyrrolderivate der Formel I stellen dagegen eine wertvolle Bereicherung des Standes der Technik dar, denn es wurde überraschend festgestellt, daß die neuen Pyrrole der Formel I ein für praktische (Freiland)-Bedürfnisse sehr günstiges Mikrobizid-Spektrum gegen phytopathogene Pilze und Bakterien aufweisen. Sie können aber nicht nur im Ackerbau oder ähnlichen Einsatzgebieten zur Bekämpfung schädlicher Mikroorganismen an Kulturpflanzen, sondern zusätzlich im Vorratsschutz zur Konservierung verderblicher Waren eingesetzt werden. Verbindungen der Formel I besitzen sehr vorteilhafte kurative, systemische und insbesondere präventive Eigenschaften und lassen sich zum Schutz von zahlreichen Kulturpflanzen, insbesondere Freilandkulturen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so z.B. gegen Cercospora, Piricularia und vor allem gegen Botrytis. Botrytis spp. (B. cinerea, B. allii) stellen mit der Grauschimmelfäule an Reben, Erdbeeren, Äpfeln, Zwiebeln und anderen Obst- und Gemüsesorten einen bedeutenden wirtschaftlichen Schadfaktor dar. Darüberhinaus lassen sich einige Verbindungen der Formel I, z.B. Verbindung Nr. 1.2, erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida.

Als Pflanzenschutzmittel besitzen die Verbindungen der Formel I ein für die praktische Anwendung in der Landwirtschaft sehr günstiges Wirkungsspektrum zum Schutze von Kulturpflanzen, ohne diese durch unerwünschte Nebenwirkungen nachteilig zu beeinflussen.

Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Darüberhinaus schließt die vorliegende Erfindung auch die Herstellung (agro)chemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen oder Vorratsgütern, das sich durch Applikation der Verbindungen der Formel I bzw. der neuen Mittel auf die Pflanzen, Pflanzenteile, deren Standort oder das Substrat kennzeichnet.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Als Vorratsschutzmittel werden die Verbindungen der Formel I in unveränderter Form oder vorzugsweise zusammen mit den in der Formulierungstechnik üblichen Hilfsmitteln eingesetzt und werden z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Gießen und die Form des Mittels werden den angestrebten Zielen und den gegebenen Verhältnissen angepaßt. Günstige Aufwandmengen liegen im allgemeinen bei 0,01 bis höchstens 2 kg Aktivsubstanz pro 100 kg zu schützendes Substrat; sie hängen jedoch ganz wesentlich von der Beschaffenheit (Größe der Oberfläche, Konsistenz, Feuchtigkeitsgehalt) des Substrats und dessen Umgebungseinflüssen ab.

Unter Lager- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenszyklus herausgenommenen

Pflanzen, deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Ertragsgüter genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Äpfel, Birnen, Pflaumen, Pfirsiche, Mandeln, Kirschen, Erd-, Him- und Brombeeren); Hülsenfrüchte (wie Bohnen, Linsen, Erbsen, Soja); Ölkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusfrüchte; Gemüsesorten (wie Spinat, Salat, Spargel, Kohlarten), Zwiebeln, Tomaten, Kartoffeln, Paprika); Lorbeergewächse (wie Avocadom Cinnamonum, Kampfer) oder Mais, Tabak, Nüsse, Kaffee, Zuckerrohr, Tee, Weintrauben, Kastanien, Hopfen, Bananen, Gras und Heu.

Als Naturprodukte tierischer Herkunft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilze (Aflatoxine und Ochratoxine) unterbunden, das Gut vor dem Verderben bewahrt und dessen Qualität für längere Zeit aufrechterhalten. Das erfindungsgemäße Verfahren kann auf alle trockenen und feuchten Vorrats- und Lagergüter angewendet werden, die gegen Mikroorganismen, wie Hefen, Bakterien und insbesondere Schimmelpilze anfällig sind.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahren ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche, Pflanze oder Substrat gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Gießen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol Ethylenglykol, Ethylenglykolmonomethyl- oder -ethylether, Ketone wie Cyclohexanon, stark polare Lösungsmittel wie N-Methyl-2-pyrrolidon,

Dimethylsulfoxid oder Dimethylformamid, sowie gegebenenfalls epoxydierte Pflanzenöle wie epoxydiertes Kokosnußöl, Sonnenblumenöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht adsorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände, wie z.B. Korkmehl oder Sägemehl, verwendet werden.

Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere Hirn, Herz, Lunge, Leber, Eidotter oder Sojabohnen gewinnen kann. Verwendbare Handelsmischungen sind z.B. Phosphatidylcholin-Mischungen. Synthetische Phospholipide sind z.B. Dioctanoylphosphatidylcholin und Dipalmititoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuß- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4–14-ethylenoxidaddukte in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 g Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seinen Nonylphenolpolyethoxyethanole, Ricinusölpolyglykolether, Polypropylen-Polyethylenoxidaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid. Auf dem Vorratsschutzgebiet werden die für die menschliche und tierische Ernährung unbedenklichen Zuschlagstoffe bevorzugt.

Die in der Formulierungstechnik gebräuchlichen Tenside sind u.a. in folgenden Publikationen beschrieben:

«Mc Cutcheon's Detergents and Emulsifiers Annual» BC Publishing Corp., Ridgewood New Jersey, 1981. Helmut Stache «Tensid-Taschenbuch» Carl Hanser-Verlag, München/Wien 1981.

Die agrochemischen Zubereitungen enthalten in der Regel 0,1 bis 99%, insbesondere 0,1 bis

95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige (agro)chemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs = absolut, wasserfrei; DMSO = Dimethylsulfoxid; DMF = Dimethylformamid. Druckangaben erfolgen in Millibar mb, oder Bar b. THF steht für Tetrahydrofuran.

Herstellungsbeispiele:

Beispiel H1:

Herstellung von

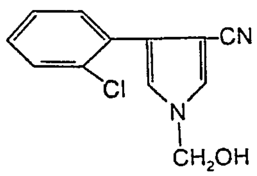

(Verb. Nr. 1.1)

N-Hydroxymethyl-3-(2-chlorphenyl)-4-cyanopyrrol

60,8 g 3-(2-Chlorphenyl)-4-cyanopyrrol, 9,9 g Paraformaldehyd und 0,8 g Triethylamin wurde gut vermischt und anschließend unter Rühren bei 90 °C Badtemperatur erwärmt. Die entstandene Schmelze wurde nach 1 h 15 min auf RT abgekühlt, wobei diese glasartig erstarrte. Die Umkristallisation aus Toluol lieferte das obige Produkt in Form bräunlicher Kristalle. Smp. 94–97 °C.

Beispiel H2:

Herstellung von

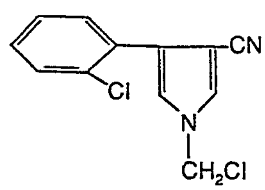

(Verb. Nr. 2.1)

N-Chlormethyl-3-(2-chlorphenyl)-4-cyanopyrrol

Zu 60 ml Thionylchlorid wurden unter kräftigem Rühren 48 g N-Hydroxymethyl-3-(2-chlorphenyl)-4-cyanopyrrol in mehreren Portionen derart zugegeben, daß eine mäßige Gasentwicklung aufrechterhalten blieb. Nach Beendigung der Gasentwicklung wurde das Gemisch noch 2 h bei RT und anschließend 2,5 h bei 35–40 °C gerührt. Nach Abkühlen auf RT wurde Toluol zugegeben und die Mischung eingeengt. Der Rückstand wurde aus Diethylether/Petroleumbenzin umkristallisiert und lieferte obiges Produkt in Form beiger Kristalle. Smp. 97–99 °C.

Beispiel H3:

Herstellung von

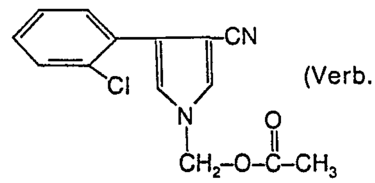

(Verb. Nr. 3.2)

N-Acetyloxymethyl-3-(2-chlorphenyl)-4-cyanopyrrol

41,4 g N-Hydroxymethyl-3-(2-Chlorphenyl)-4-cyanopyrrol wurden in 350 ml Pyridin gelöst und mit 1,8 g 4-Dimethylaminopyridin versetzt. Anschließend wurden bei 0° bis 7 °C 21,4 ml Essigsäureanhydrid langsam zugetropft und das Gemisch bei 0° bis 7 °C 12 h gerührt; dann wurde das Reaktionsgemisch auf Eiswasser gegossen und zweimal mit Essigsäureethylester extrahiert. Die Extrakte wurden zweimal mit verdünnter, eiskalter Salzsäure und zweimal mit halbgesättigter, wäßriger Natriumchlorid-Lösung gewaschen, über Natriumsulfat getrocknet, filtriert und das Filtrat eingeengt. Der Rückstand wurde aus Diethylether/Hexan umkristallisiert, wobei das obige Produkt in Form von farblosen Kristallen anfiel. Smp. 91–95 °C.

Beispiel H4:

Herstellung von

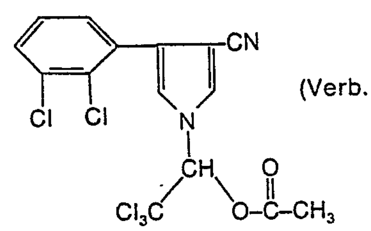

(Verb. Nr. 3.4)

N-(1-Acetyloxy-2,2,2-trichlorethyl)-3-(2,3-dichlorphenyl)-4-cyanopyrrol

Zu 2,4 g 3-(2,3-Dichlorphenyl)-4-cyanopyrrol in 50 ml THF wurden nacheinander 1,2 ml Chloral und 1,8 ml Triethylamin getropft. Das Reaktionsgemisch wurde 1,5 h bei 25 °C gerührt, dann mit 0,1 g 4-Dimethylaminopyridin versetzt und auf −10 °C abgekühlt. Bei −10° bis −5 °C wurde nun eine Lösung von 0,9 ml Acetylchlorid in 10 ml THF sehr langsam zugetropft und das Reaktionsgemisch anschließend 30 min bei 0° und dann 1 h bei RT gerührt, filtriert und das Filtrat eingeengt. Der Rückstand wurde in Diethylether gelöst, die Etherlösung zweimal mit halbgesättigter Natriumchlorid-Lösung gewaschen, über Natriumsulfat getrocknet, filtriert und das Filtrat eingeengt. Das Rohprodukt wurde aus Diethylether/Petroleumbenzin umkristallisiert und lieferte farblose Kristalle des obigen Produkts. Smp. 111–113 °C.

Beispiel H5:
Herstellung von

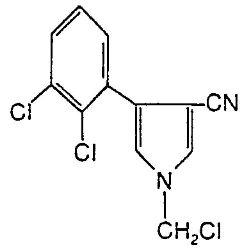

(Verb. Nr. 2.4)

N-Chlormethyl-3-(2,3-dichlorphenyl)-4-cyanopyrrol

Zu 23,7 g 3-(2,3-Dichlorphenyl)-4-cyanopyrrol gelöst in 300 ml THF werden 7,5 g Paraformaldehyd und anschließend 1,5 und 1,8-Diazabicyclo[5,5,0]undec-7-en gegeben. Es wird während 3 h bei RT gerührt. Anschließend werden unter Rühren 21,7 ml Thionylchlorid bei 20° bis 30 °C zum entstehenden Hydroxymethyl-Derivat getropft. Das Reaktionsgemisch wird während 16 h bei RT gerührt, anschließend auf Eiswasser gegossen und das resultierende Gemisch zweimal mit Essigsäureethylester extrahiert. Die vereinigten organischen Extrakte werden noch zweimal mit halbgesättigter Natriumchlorid-Lösung gewaschen, über Natriumsulfat getrocknet, filtriert und eingeengt. Kristallisation des Rückstandes aus Ether/Petroleumbenzin liefert das obige Produkt in Form beiger Kristalle. Smp. 132–133 °C.

Beispiel H6:
Herstellung von

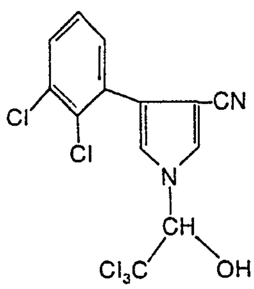

(Verb. Nr. 1.3)

N-(1-Hydroxy-2,2,2-trichlorethyl)-3-(2,3-dichlorphenyl)-4-cyanopyrrol

Zu 64,1 g 3-Cyano-4-(2,3-Dichlorphenyl)-pyrrol und 1,2 ml Triethylamin in 400 ml THF werden bei 20° bis 29 °C 39,6 ml Chloral zugetropft. Anschließend werden nochmals 2,4 ml Triethylamin und dann 1,2 ml Diazabicyclo[5,4,0]undec-7-en zugegeben und die klare Lösung zuerst 2 h bei RT, dann 14 h bei 0° bis 5 °C gerührt. Die Lösung wird dann auf verdünnte Salzsäure und Eis gegossen und das Gemisch zweimal mit Essigsäureethylester extrahiert. Die organischen Extrakte werden dreimal mit kalter, halbgesättigter Natriumchlorid-Lösung gewaschen, über Natriumsulfat getrocknet filtriert und eingeengt. Kristallisation des Rückstandes aus Ether/Petroleumether lieferte das gewünschte Produkt als farblose Kristalle. Smp. 117 °C (Zers).

Beispiel H7:
Herstellung von

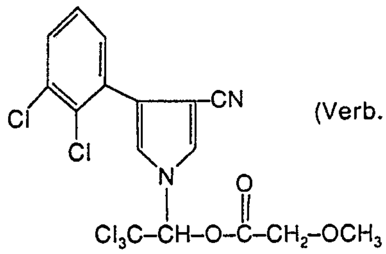

(Verb. Nr. 3.5)

N-[1-(Methoxyacetyloxy)-2,2,2-trichlorethyl]-3-(2,3-dichlorphenyl)-4-cyanopyrrol

4,8 g 3-Cyano-4-(2,3-Dichlorphenyl)-pyrrol werden in 50 ml Tetrahydrofuran gelöst und bei Raumtemperatur mit 0,2 ml DBU dann 2,3 ml Chloral versetzt. Die Lösung wird auf −5 °C abgekühlt, dann werden 3,3 ml Triethylamin zugetropft. Nach 1 Stunde Rühren bei −5 °C werden bei −10° bis −5 °C langsam 2,2 ml Methoxyessigsäurechlorid, gelöst in 10 ml THF, zugetropft. Nach 1 h Rühren bei auftauendem Kühlbad wird auf Eiswasser gegossen und das Gemisch zweimal mit Essigsäureethylester extrahiert. Die organischen Extrakte werden zweimal mit kalter verdünnter Salzsäure und zweimal mit kalter halbgesättigter Natriumchlorid-Lösung gewaschen, über Natriumsulfat getrocknet, filtriert und eingeengt. Kristallisation des Rückstandes aus Petroleumbenzin liefert das obige Produkt als farblose Kristalle. Smp. 88–90 °C.

Beispiel H8:
Herstellung von

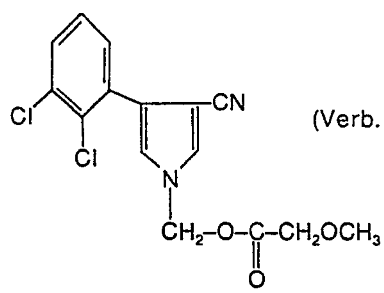

(Verb. Nr. 3.22)

N-[(Methoxyacetyloxy)methyl]-3-(2,3-dichlorphenyl)-4-cyanopyrrol

4,5 g 3-Cyano-4-(2,3-Dichlorphenyl)-pyrrol werden in 50 ml Tetrahydrofuran gelöst und mit 0,65 g Paraformaldehyd, 3,0 ml Triethylamin und 0,5 ml DBU versetzt. Das Gemisch wird 2 h bei RT gerührt wobei eine klare Lösung entsteht. Anschließend werden unter Rühren 2,0 ml Äthoxyessigsäurechlorid bei 5 °C langsam zugetropft. Nach weiteren 2,5 h bei 0° bis 5 °C wird filtriert und das Filtrat eingeengt. Kristallisation des Rückstandes aus Ether/Petroleumbenzin liefert das obige Produkt als farblose Kristalle. Smp. 72–75 °C.

Auf analoge Weise lassen sich auch die Verbindungen der nachfolgenden Tabellen herstellen:

Tabelle 1:
Verbindungen der Formel

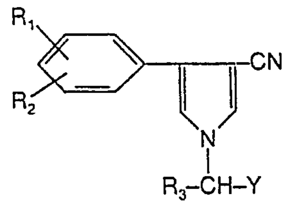

worin Y für OH steht.

| Verb. Nr. | $R_1$ | $R_2$ | $R_3$ | Physik. Konst. |
|---|---|---|---|---|
| 1.1 | 2–Cl | H | H | Smp. 94–97° |
| 1.2 | H | 3–Cl | H | Smp. 72–74° |
| 1.3 | 2–Cl | 3–Cl | $CCl_3$ | 117° Zers. |
| 1.4 | H | 2–$SCH_3$ | $CCl_3$ | 85° Zers. |
| 1.5 | 2–Cl | H | $CCl_3$ | 70–76° Zers. |
| 1.6 | 2–Cl | 3–Cl | H | Smp. 137–140° |
| 1.7 | H | 3–Cl | $CCl_3$ | - |
| 1.8 | 2–$SCH_3$ | H | H | |
| 1.9 | 2–$OCH_3$ | H | H | Harz |
| 1.10 | 2–$OCH_3$ | H | $CCl_3$ | |
| 1.11 | H | H | H | Smp. 81–84° |
| 1.12 | H | 3–$OCH_3$ | H | |
| 1.13 | H | 3–$OCH_3$ | $CCl_3$ | Harz |
| 1.14 | H | 3–$SCH_3$ | $CCl_3$ | |
| 1.15 | H | 3–$SCH_3$ | H | Harz |
| 1.16 | 2–$OCH_3$ | 3–$OCH_3$ | H | |
| 1.17 | 2–$OCH_3$ | 3–$OCH_3$ | $CCl_3$ | |
| 1.18 | H | 3–Br | H | Smp. 65–69° |
| 1.19 | H | 3–Br | $CCl_3$ | |
| 1.20 | H | 3–F | H | Smp. 75–77° |
| 1.21 | 2–F | H | H | |
| 1.22 | H | 3–F | $CCl_3$ | |
| 1.23 | H | 3–J | $CCl_3$ | |
| 1.24 | H | 3–J | H | |

Tabelle 2:
Verbindungen der Formel

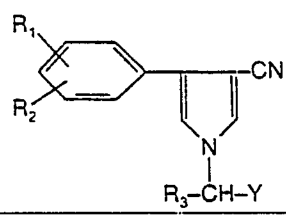

worin Y für Cl steht.

| Verb. Nr. | $R_1$ | $R_2$ | $R_3$ | Physik. Konst. |
|---|---|---|---|---|
| 2.1 | 2–Cl | H | H | Smp. 97–99° |
| 2.2 | H | 3–Cl | H | Smp. 77–79° |
| 2.3 | 2–Cl | H | $CCl_3$ | |
| 2.4 | 2–Cl | 3–Cl | H | Smp. 132–133° |
| 2.5 | 2–Cl | 3–Cl | $CCl_3$ | Smp. 152–157° |
| 2.6 | H | 3–Cl | $CCl_3$ | |
| 2.7 | 2–$SCH_3$ | H | H | |
| 2.8 | 2–$SCH_3$ | H | $CCl_3$ | |
| 2.9 | 2–$OCH_3$ | H | H | |
| 2.10 | 2–$OCH_3$ | H | $CCl_3$ | |
| 2.11 | H | 3–$OCH_3$ | H | Harz |
| 2.12 | H | 3–$OCH_3$ | $CCl_3$ | Harz |
| 2.13 | H | 3–$SCH_3$ | H | |
| 2.14 | H | 3–$SCH_3$ | $CCl_3$ | |
| 2.15 | 2–$OCH_3$ | 3–$OCH_3$ | H | |
| 2.16 | 2–$OCH_3$ | 3–$OCH_3$ | $CCl_3$ | |
| 2.17 | H | 3–Br | H | Smp. 71–74° |
| 2.18 | H | 3–Br | $CCl_3$ | semikristallin |

Tabelle 2: (Fortsetzung)

| Verb. Nr. | $R_1$ | $R_2$ | $R_3$ | Physik. Konst. |
|---|---|---|---|---|
| 2.19 | H | 3–F | H | Smp. 80–83° |
| 2.20 | 2–F | H | H | |
| 2.21 | H | 3–F | $CCl_3$ | |
| 2.22 | H | 3–J | $CCl_3$ | |
| 2.23 | H | 3–J | H | Smp. 59–65° |
| 2.24 | H | 3–$OCH_3$ | $CHCl_2$ | |
| 2.25 | H | 3–$OCH_3$ | $CHCl_2$ | Harz |
| 2.26 | H | 3–$SCH_3$ | $CH_2Cl$ | |

Tabelle 3:
Verbindungen der Formel

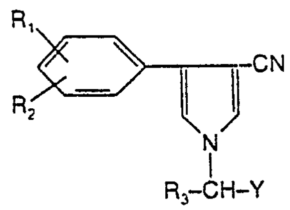

worin Y für $-OC(O)R_4$ steht.

| Verb. Nr. | $R_1$ | $R_2$ | $R_3$ | $R_4$ | Physik. Konst. |
|---|---|---|---|---|---|
| 3.1 | 2–Cl | H | $CCl_3$ | $CH_3$ | Smp. 100–108° |
| 3.2 | 2–Cl | H | H | $CH_3$ | Smp. 91–95° |
| 3.3 | H | 3–Cl | $CCl_3$ | $CH_3$ | Smp. 118–120° |
| 3.4 | 2–Cl | 3–Cl | $CCl_3$ | $CH_3$ | Smp. 111–113° |
| 3.5 | 2–Cl | 3–Cl | $CCl_3$ | $CH_2OCH_3$ | Smp. 88–90° |
| 3.6 | 2–$SCH_3$ | H | $CCl_3$ | $CH_3$ | Smp. 139–141° |
| 3.7 | 2–$OCH_3$ | H | $CCl_3$ | $CH_3$ | Smp. 106–108° |
| 3.8 | 2–Cl | H | H | $CCl_3$ | |
| 3.9 | 2–Cl | H | H | $CH_2OCH_3$ | Smp. 112–114° |
| 3.10 | H | 3–Cl | $CCl_3$ | $CH(CH_3)_2$ | |
| 3.11 | H | 3–Cl | $CCl_3$ | $(CH_2)_3CH_3$ | Harz |
| 3.12 | H | 3–Cl | $CCl_3$ | $CH_2Br$ | |
| 3.13 | H | 3–Cl | $CCl_3$ | $CF_3$ | |
| 3.14 | H | 3–Cl | H | $CH_3$ | |
| 3.15 | H | 3–Cl | H | $CHCl_2$ | |
| 3.16 | 2–Cl | 3–Cl | $CCl_3$ | $C_2H_5$ | Smp. 137–141° |
| 3.17 | 2–Cl | 3–Cl | $CCl_3$ | $(CH_2)_3CH_3$ | $n_D^{50}$ 1.5548 |
| 3.18 | 2–Cl | 3–Cl | $CCl_3$ | $CH_2Cl$ | Smp. 143–151° |
| 3.19 | 2–Cl | 3–Cl | $CCl_3$ | H | Harz |
| 3.20 | 2–Cl | 3–Cl | H | $CH_3$ | Smp. 95–99° |
| 3.21 | 2–Cl | 3–Cl | H | $C(CH_3)_3$ | Smp. 86–88° |
| 3.22 | 2–Cl | 3–Cl | H | $CH_3OCH_3$ | Smp. 72–75° |
| 3.23 | 2–Cl | 3–Cl | H | $CCl_3$ | |
| 3.24 | 2–Cl | 3–Cl | H | H | |
| 3.25 | 2–$OCH_3$ | H | $CCl_3$ | $C(CH_3)_3$ | |
| 3.26 | 2–$OCH_3$ | H | $CCl_3$ | $CH_2Cl$ | Harz |
| 3.27 | 2–$OCH_3$ | H | $CCl_3$ | H | |
| 3.28 | 2–$OCH_3$ | H | H | $CCl_3$ | |
| 3.29 | 2–$OCH_3$ | H | H | $CH_3$ | |
| 3.30 | 2–$OCH_3$ | H | H | $CH_2OCH_3$ | semikristallin |
| 3.31 | 2–$SCH_3$ | H | $CCl_3$ | $C_2H_5$ | |
| 3.32 | 2–$SCH_3$ | H | $CCl_3$ | $CH_2Cl$ | |
| 3.33 | 2–$SCH_3$ | H | $CCl_3$ | $CH_2OCH_3$ | semikristallin |
| 3.34 | 2–$SCH_3$ | H | H | $CH_3$ | |
| 3.35 | 2–$SCH_3$ | H | H | $C_2H_5$ | |
| 3.36 | 2–$SCH_3$ | H | H | $CCl_3$ | |
| 3.37 | 2–Cl | 3–Cl | H | $CH_2Cl$ | Smp. 113–115° |
| 3.38 | 2–Cl | 3–Cl | H | $C_2H_5$ | Harz |
| 3.39 | 2–Cl | 3–Cl | H | $C_4H_9$–n | $n_D^{50}$ 1.5571 |

Tabelle 3 (Fortsetzung)

| Verb. Nr. | $R_1$ | $R_2$ | $R_3$ | $R_4$ | Physik. Konst. |
|---|---|---|---|---|---|
| 3.40 | 2–Cl | 3–Cl | $CCl_3$ | $C(CH_3)_3$ | Smp. 146–147° |
| 3.41 | H | H | $CCl_3$ | $CH_2OCH_3$ | Smp. 121–122° |
| 3.42 | 2–Cl | 3–Cl | $CCl_3$ | $C_2H_5$ | semikristallin |
| 3.43 | 2–Cl | 3–Cl | H | $CH_2Br$ | Smp. 88–90° |
| 3.44 | 2–Cl | 3–Cl | $CCl_3$ | $CH_2Br$ | Smp. 132–135° |
| 3.45 | 2–Cl | 3–Cl | $CCl_3$ | $CH_2OC_2H_5$ | Smp. 115–117° |
| 3.46 | 2–Cl | 3–Cl | H | $CH_2OC_2H_5$ | Smp. 83–85° |
| 3.47 | 2–Cl | H | $CCl_3$ | $CH_2OCH_3$ | Smp. 116–118° |
| 3.48 | H | H | H | $CH_2OCH_3$ | Smp. 78–79° |
| 3.49 | 2–Cl | 3–Cl | H | $CH_2OC_3H_7$–n | Smp. 87–88° |
| 3.50 | 2–Cl | 3–Cl | $CCl_3$ | $CH_2OC_3H_7$–n | Smp. 76–78° |
| 3.51 | 2–Cl | 3–Cl | H | $CH_2OC_4H_9$–n | Smp. 71–74° |
| 3.52 | 2–Cl | 3–Cl | $CCl_3$ | $CH_2OC_4H_9$–n | Smp. 89–91° |
| 3.53 | 2–Cl | 3–Cl | H | $CH_2OC_6H_{13}$–n | |
| 3.54 | 2–Cl | 3–Cl | $CCl_3$ | $CH_2OC_6H_{13}$–n | |
| 3.55 | 2–Cl | H | H | $CH_3OCH(CH_3)_2$ | |
| 3.56 | 2–Cl | H | $CCl_3$ | $CH_2OCH(CH_3)_2$ | Smp. 69–76° |
| 3.57 | 2–Cl | 3–Cl | H | $CH_2OCH(CH_3)_2$ | Smp. 74–75° |
| 3.58 | 2–Cl | 3–Cl | $CCl_3$ | $CH_2OCH(CH_3)_2$ | Smp. 112–115° |
| 3.59 | 2–Cl | 3–Cl | H | $CH_2OCH(CH_3)C_2H_5$ | Smp. 71–73° |
| 3.60 | 2–Cl | 3–Cl | $CCl_3$ | $CH_2OCH(CH_3)C_2H_5$ | Smp. 70–74° |
| 3.61 | 2–Cl | 3–Cl | H | $CH_2OC(CH_3)_3$ | |
| 3.62 | 2–Cl | 3–Cl | $CCl_3$ | $CH_2OC(CH_3)_3$ | |
| 3.63 | 2–Cl | H | H | $CH_2OCH_2CH_2OCH_3$ | |
| 3.64 | 2–Cl | H | $CCl_3$ | $CH_2OCH_2CH_2OCH_3$ | |
| 3.65 | 2–Cl | 3–Cl | H | $CH_2OCH_2CH_2OCH_3$ | Smp. 99–101° |
| 3.66 | 2–Cl | 3–Cl | $CCl_3$ | $CH_2OCH_2CH_2OCH_3$ | $n_D^{50}$ 1.5472 |
| 3.67 | 2–Cl | 3–Cl | H | $CH_2OCH_2CH=CH_2$ | Smp. 65–67° |
| 3.68 | 2–Cl | 3–Cl | $CCl_3$ | $CH_2OCH_2CH=CH_2$ | Smp. 86–88° |
| 3.69 | 2–Cl | 3–Cl | H | $CH_2OCH_2C\equiv CH$ | Smp. 109–111° |
| 3.70 | 2–Cl | 3–Cl | $CCl_3$ | $CH_2OCH_2C\equiv CH$ | $n_D^{50}$ 1.5732 |
| 3.71 | 2–Cl | 3–Cl | H | $CH_3OC_6H_5$ | Smp. 119–121° |
| 3.72 | 2–Cl | 3–Cl | $CCl_3$ | $CH_2OC_6H_5$ | $n_D^{50}$ 1.5803 |
| 3.73 | 2–Cl | 3–Cl | H | $CH_2OC_6H_3Cl_2$(3,5) | Smp. 125–127° |
| 3.74 | 2–Cl | 3–Cl | $CCl_3$ | $CH_2OC_6H_3Cl_2$(3,5) | Smp. 154–156° |
| 3.75 | 2–Cl | 3–Cl | H | $CH_2OC_6H_3Cl_2$(2,4) | Smp. 102–104° |
| 3.76 | 2–Cl | 3–Cl | $CCl_3$ | $CH_2OC_6H_3Cl_2$(2,4) | Smp. 157–159° |
| 3.77 | 2–Cl | H | H | 2–Tetrahydrofuryl | $n_D^{50}$ 1.5672 |
| 3.78 | 2–Cl | H | $CCl_3$ | 2–Tetrahydrofuryl | Smp. 142–149° |
| 3.79 | 2–Cl | 3–Cl | H | 2–Tetrahydrofuryl | Smp. 96–98° |
| 3.80 | 2–Cl | 3–Cl | $CCl_3$ | 2–Tetrahydrofuryl | Smp. 156–160° |
| 3.81 | 2–Cl | H | H | $CH_2SCH_3$ | |
| 3.82 | 2–Cl | H | $CCl_3$ | $CH_2SCH_3$ | |
| 3.83 | H | H | H | $CH_2SCH_3$ | |
| 3.84 | H | H | $CCl_3$ | $CH_2SCH_3$ | |
| 3.85 | 2–Cl | 3–Cl | H | $CH_2SCH_3$ | |
| 3.86 | 2–Cl | 3–Cl | $CCl_3$ | $CH_2SCH_3$ | |
| 3.87 | 2–Cl | 3–Cl | H | $CH_2SCH(CH_3)C_2H_5$ | Harz |
| 3.88 | 2–Cl | 3–Cl | $CCl_3$ | $CH_2SCH(CH_3)C_2H_5$ | |
| 3.89 | 2–Cl | H | H | $CH_2SCH(CH_3)C_2H_5$ | |
| 3.90 | 2–Cl | H | $CCl_3$ | $CH_2SCH(CH_3)C_2H_5$ | |
| 3.91 | 2–Cl | 3–Cl | H | $CH_2SC_4H_9$–n | |
| 3.92 | 2–Cl | 3–Cl | $CCl_3$ | $CH_2SC_4H_9$–n | |
| 3.93 | 2–Cl | 3–Cl | H | $CH_2SC(CH_3)_3$ | |
| 3.94 | 2–Cl | 3–Cl | $CCl_3$ | $CH_2SC(CH_3)_3$ | |
| 3.95 | 2–Cl | 3–Cl | H | $C(O)OC_2H_5$ | Smp. 111–113° |
| 3.96 | 2–Cl | H | H | $CH_2SC_6H_5$ | |
| 3.97 | 2–Cl | H | $CCl_3$ | $CH_2SC_6H_5$ | |
| 3.98 | 2–Cl | 3–Cl | H | $CH_2SC_6H_5$ | |
| 3.99 | 2–Cl | 3–Cl | $CCl_3$ | $CH_2SC_6H_5$ | |

Tabelle 3 (Fortsetzung)

| Verb. Nr. | $R_1$ | $R_2$ | $R_3$ | $R_4$ | Physik. Konst. |
|---|---|---|---|---|---|
| 3.100 | 2–Cl | H | H | $CH=CH_2$ | |
| 3.101 | 2–Cl | H | $CCl_3$ | $CH=CH_2$ | |
| 3.102 | 2–Cl | 3–Cl | H | $CH=CH_2$ | |
| 3.103 | 2–Cl | 3–Cl | $CCl_3$ | $CH=CH_2$ | Smp. 106–108° |
| 3.104 | 2–Cl | 3–Cl | H | $CH=CH-CH_3$ | |
| 3.105 | 2–Cl | 3–Cl | $CCl_3$ | $CH=CH-CH_3$ | Smp. 107–109° |
| 3.106 | 2–Cl | H | H | $CH=CH-CH_3$ | |
| 3.107 | 2–Cl | H | $CCl_3$ | $CH=CH-CH_3$ | |
| 3.108 | H | H | H | $CH_2Cl$ | Smp. 83–84° |
| 3.109 | H | 3–$OCH_3$ | H | $CH_3$ | |
| 3.110 | H | 3–$OCH_3$ | $CCl_3$ | $CH_3$ | |
| 3.111 | H | 3–$OCH_3$ | $CCl_3$ | $CH_3$ | |
| 3.112 | H | 3–$OCH_3$ | H | $CH_3$ | Harz |
| 3.113 | 2–$OCH_3$ | 3–$OCH_3$ | H | $CH_2OCH_3$ | Harz |
| 3.114 | 2–$OCH_3$ | 3–$OCH_3$ | H | $CH_3$ | |
| 3.115 | H | 3–Br | $CCl_3$ | $CH_2OCH_3$ | |
| 3.116 | H | 3–Br | H | $CH_3$ | |
| 3.117 | H | 3–F | H | $CH_3$ | |
| 3.118 | 2–F | H | H | $CH_2OCH_3$ | |
| 3.119 | H | 3–F | $CCl_3$ | $CH_3$ | |

Formulierungsbeispiele für flüssige Wirkstoffe der Formel I
(% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | – | – |
| Tributylphenoyl-polyethylenglykolether (30 Mol Ethylenoxid) | – | 12% | 4% |
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | – | – | – |
| Polyethylenglykol MG 400 | – | 70% | – | – |
| N-Methyl-2-pyrrolidon | – | 20% | – | – |
| Epoxydiertes Kokosnußöl | – | – | 1% | 5% |
| Benzin (Siedegrenzen 160–190 °C) | – | – | 94% | – |

(MG = Molekulargewicht)

| F3. Granulate | a) | b) |
|---|---|---|
| Wirkstoff aus den Tabellen | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | – |
| Kaolin | – | 90% |

Durch inniges Vermischen auf Trägerstoffe mit dem Wirkstoff erhält man gebrauchsfertige Stäubemittel.

Formulierungsbeispiele für feste Wirkstoffe der Formel I
(% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyethylenglykolether (7–8 Mol Ethylenoxid) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F6. Emulsions-Konzentrat | |
|---|---|
| Wirkstoff aus den Tabellen | 10% |
| Octylphenolpolyethylenglykolether (4–5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | – |
| Kaolin | – | 92% |

Man erhält anwendungsfertige Stäubemittel, indem der Wirkstoff mit den Träger vermischt auf einer geeigneten Mühle vermahlen wird.

| F8. Extruder Granulat | |
|---|---|
| Wirkstoff aus den Tabellen | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschließend im Luftstrom getrocknet.

| F9. Umhüllungs-Granulat | |
|---|---|
| Wirkstoff aus den Tabellen | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| F10. Suspensions-Konzentrat | |
|---|---|
| Wirkstoff aus den Tabellen | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wäßrige Formaldehyd-Lösung | 0,2% |
| Silikonöl in Form einer 75%igen wäßrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:
Beispiel B1:
Wirkung gegen Puccinia graminis auf Weizen
a) Residual-protektive Wirkung

Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus den Tabellen 1 bis 3 zeigten nicht nur im obigen Gewächshausversuch sondern auch im Freiland gegen Puccinia-Pilze eine gute Wirkung. Unbehandelte aber infizierte Kontrollpflanzen zeigten einen Puccinia-Befall von 100%. Unter anderen hemmten die Verbindungen Nr. 1.2 bis 1.5, 1.9, 1.13, 2.11, 2.12, 2.18, 3.2, 3.4 bis 3.7, 3.37 bis 3.40, 3.42 bis 3.46, 3.51, 3.52, 3.57, 3.70, 3.73 bis 3.76 und 3.94 den Puccinia-Befall auf 0 bis 10%.

Beispiel B2:
Wirkung gegen Cercospora arachidicola auf Erdnußpflanzen

a) Residual-protektive Wirkung

10–15 cm hohe Erdnußpflanzen wurden mit einer aus Spritzpulver der Wirksubstanz hergestellten Spritzbrühe (0,006% Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschließend bis zum Auftreten der typischen Blattflecken in einem Gewächshaus aufgestellt. Die Beurteilung der fungiziden Wirkung erfolgt 12 Tage nach der Infektion basierend auf Anzahl und Größe der auftretenden Flecken.

b) Systemische Wirkung

Zu 10–15 cm hohen Erdnußpflanzen wurde eine aus Spritzpulver des Wirkstoffes hergestellte Spritzbrühe gegossen (0,06% Aktivsubstanz bezogen auf das Erdvolumen). Nach 48 Stunden wurden die behandelten Pflanzen mit einer Konidiensuspension des Pilzes infiziert und 72 Stunden bei ca. 21 °C und hoher Luftfeuchtigkeit inkubiert. Anschließend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Größe der Flecken = 100%), zeigten Erdnußpflanzen, die im Gewächshaus oder im Freiland mit Wirkstoffen aus den Tabellen 1 bis 3 behandelt wurden, einen stark reduzierten Cercospora-Befall. So verhinderten die Verbindungen 1.3, 1.6, 3.4, 3.5, 3.17, 3.18, 3.20, 3.49, 3.51, 3.52, 3.57, 3.67, 3.69, 3.70, 3.75 und 3.95 den Cercosporabefall fast vollständig (8%).

Beispiel B3:
Wirkung gegen Botrytis cinerea auf Bohnen
Residual protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95–100% relativer Luftfeuchtigkeit und 21 °C erfolgte die Beurteilung des Pilzbefalls. Die Verbindungen aus den Tabellen 1 bis 3 hemmten nicht nur in obigem Modellversuch sondern auch im Freiland die Pilzinfektion sehr stark. Bei einer Konzentration von 0,02% erwiesen sich z. B. die Verbindungen Nr. 1.1 bis 1.6, 1.9, 1.11, 1.13, 1.15, 1.18, 1.20, 2.1, 2.2, 2.4, 2.5, 2.11, 2.12, 2.17, 2.18, 2.19, 2.23, 3.1 bis 3.7, 3.9, 3.16 bis 3.22, 3.26, 3.30, 3.33, 3.37, 3.38, 3.39 bis 3.52, 3.56 bis 3.60, 3.65 bis 3.80, 3.95, 3.103, 3.105, 3.108, 3.112 und 3.113 als voll wirksam (Krankheitsbefall 0 bis 5%). Einige Vertreter zeigten diese Wirkung auch bei der halben Aufwandmenge. Der Befall unbehandelter aber infizierter Bohnenpflanzen betrug 100%.

Beispiel B4:
Wirkung gegen Botrytis cinerea an Apfelfrüchten

Künstlich verletzte Äpfel wurden behandelt, indem eine aus Spritzpulver der Wirksubstanz hergestellte Spritzbrühe auf die Verletzungsstellen aufgetropft wurde. Die behandelten Früchte wurden anschließend mit einer Sporensuspension von Botrytis cinerea inokuliert und während einer Woche bei einer hohen Luftfeuchtigkeit und ca. 20 °C inkubiert.

Bei der Auswertung wurden die angefaulten Verletzungsstellen gezählt und daraus die fungizide Wirkung der Testsubstanz abgeleitet. Unter anderen hemmten die Verbindungen Nr. 1.1 bis 1.6, 1.9, 1.11, 1.13, 1.15, 1.18, 1.20, 2.1, 2.2, 2.4, 2.5, 2.11, 2.12, 2.17, 2.18, 2.19, 2.23, 3.1 bis 3.7, 3.9, 3.16 bis 3.22, 3.26, 3.30, 3.33, 3.37, 3.38, 3.39 bis 3.52, 3.56 bis 3.60, 3.65 bis 3.80, 3.95, 3.103, 3.105, 3.108 3.112 und 3.113 den Pilzbefall im Vergleich zu unbehandelten Kontrollfrüchten (100% Befall) fast vollständig.

Beispiel B5:
Wirkung gegen Piricularia oryzae auf Reis

Reispflanzen wurden nach zweiwöchiger Anzucht mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95–100% relativer Luftfeuchtigkeit und 24 °C wurde der Pilzbefall beurteilt. Verbindungen der Formel I zeigten eine gute Hemmung des Piriculariabefalls, so z. B. die Verbindungen Nr. 1.3, 1.6, 2.2, 2.4, 3.1 bis 3.5, 3.7, 3.17, 3.18, 3.20, 3.21, 3.44 bis 3.47, 3.49, 3.51, 3.52, 3.57, 3.67, 3.70, 3.75 und 3.95; sie reduzierten den Befall auf weniger als 10%, wobei diese Wirkung auch unter Freilandbedingungen erzielt werden konnte.

Beispiel B6:
Wirkung gegen Rhizoctonia solani auf Kohl
Wirkung nach Bodenapplikation

Der Pilz wurde auf sterilen Hirsekörnern kultiviert und einer Erde-Sand-Mischung beigegeben. Die infizierte Erde wurde in Schalen abgefüllt und mit Kohlsamen besät. Gleich nach der Aussaat wurde das als Spritzpulver formulierte Versuchspräparat als wäßrige Suspension über die Erde gegossen (20 ppm Aktivsubstanz bezogen auf das Erdvolumen). Die Erdschalen wurden darauf 2–3 Wochen bei ca. 24 °C im Gewächshaus aufgestellt und durch leichtes Überbrausen stets gleichmäßig feucht gehalten. Bei der Auswertung des Tests wurde der Auflauf der Kohlpflanzen bestimmt.

Nach Behandlung mit Spritzpulvern, die als Aktivsubstanz eine der Verbindungen Nr. 1.3, 2.1, 3.20 oder 3.37 enthielten, liefen über 80% der Kohlsamen auf, und die Pflanzen hatten ein gesundes Aussehen.

Beispiel B7:
Residual protektive Wirkung gegen Venturia inaequalis auf Apfeltrieben

Apfelstecklinge mit 10–12 cm langen Frischtrieben wurden mit einer aus Spritzpulver des Wirkstoffes hergestellten Spritzbrühe (0,006% Aktivsubstanz) besprüht. Nach 24 Stunden wurden die behandelten Pflanzen mit einer Konidiensuspension des Pilzes infiziert. Die Pflanzen wurden dann während 5 Tagen bei 90–100% relativer Luftfeuchtigkeit inkubiert und während 10 weiteren Tagen in einem Gewächshaus bei 20–24 °C aufgestellt. Der Schorfbefall wurde 15 Tage nach der Infektion beurteilt. Verbindungen der Formel I zeigten eine gute Wirkung gegen Venturia-Erreger, insbesondere die Verbindungen Nr. 1.2, 1.3, 3.4, 3.5, 3.17, 3.18, 3.20, 3.22, 3.37, 3.39, 3.71 und 3.95 hemmten den Schorfbefall im Vergleich zu unbehandelten Kontrollpflanzen auf weniger als 20%. Die Verbindungen Nr. 1.3, 3.4, 3.5 und 3.17 erreichten dies im Freiland sogar noch in einer Dosierung von 0,002%.

Beispiel B8:
Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Größe der Pilzkolonien werden zur Beurteilung der Testprodukte herangezogen. Dabei verhinderten die Verbindungen aus den Tabellen 1 bis 3 insbesondere aus Tabelle 3 den Pilzbefall weitgehend (0 bis 10%).

Beispiel B9:
Wirkung gegen Fusarium nivale

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Größe der Pilzkolonien werden zur Beurteilung der Testprodukte herangezogen.

Bei den Körnern, die mit einem Spritzpulver behandelt worden sind, das als Wirkstoff eine der Verbindungen aus den Tabellen 1 bis 3 enthielt, wurde die Entwicklung der Pilzkolonien fast vollständig unterdrückt (0 bis 5%).

Beispiel B10:
Körnerschutz: (Grainpreservative Test)
a) Kurzzeittest gegen Schimmelpilze an Feucht-Mais

Trockene, als Tierfutter vorgesehene Maiskörner (Portionen à 80 g) werden in verschließbaren Plastikbechern mit Wirkstoffen aus den Tabellen 1 bis 3 in Form einer wäßrigen Suspension, Emulsion oder Lösung gut durchmischt. Die Substanzapplikation wird so bemessen, daß eine Konzentration von 0,06% AS bezüglich des Maistrockengewichts erreicht wird. Ein befeuchteter Papierlappen sorgt für eine feuchtigkeitsgesättigte Atmosphäre in den mit Mais gefüllten und anschließend verschlossenen Bechern. Nach 2–3 Wochen Inkubation bei ca. 20 °C entwickelt sich bei den nur mit Wasser behandelten Mais-Proben spontan eine Mischpopulation von Schimmelpilzen. Eine künstliche Infektion erübrigt sich. Das Ausmaß der Pilzentwicklung nach 3 Wochen dient zur Bewertung der Wirksamkeit der Verbindungen der Formel I.

b) Langzeittest gegen Schimmelpilze an Feucht-Mais
Pilzbefall aufweisen, werden während zwei weiteren Monaten inkubiert. Nach jedem Monat erfolgt eine visuelle Bonitur nach denselben Kriterien wie im a-Test.

II. Die Testdurchführung erfolgt grundsätzlich gleich wie unter a und b, doch wird die Testsubstanz bei 2000, 600 und 200 ppm AS (bezogen auf das Maistrockengewicht) während 6 Monaten geprüft.

Durch Behandlung mit Verbindungen der Formel I aus den Tabellen 1 bis 3 wurde die Bildung von Schimmelpilzen an Feuchtmais in allen drei Tests a, bI und bII sowohl kurzfristig (3 Wochen) als auch langfristig (6 Monate) vollständig unterdrückt.

So verhinderten z.B. die Verbindungen 1.1 bis 1.6, 1.9, 1.11, 1.13, 1.15, 1.18 und 1.20 sowie einige Vertreter aus den Tabellen 2 und 3 in allen drei Tests a, bI und bII bei einer Prüfkonzentration von 600 ppm AS den Schimmelpilzbefall fast vollständig (0–5% Befall).

In ähnlichen Versuchen, bei denen anstatt Futtermais wahlweise Futtergetreide (Hafer), Heu, Rübenschnitzel oder Saubohnen verwendet wurden, beobachtete man gleichartige Resultate eines mehrmonatigen Dauerschutzes bei Behandlung mit obengenannten Wirksubstanzen der Formel I.

**Patentansprüche**

1. Verbindungen der Formel I

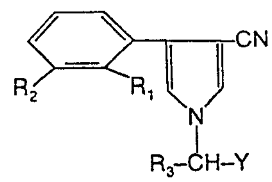

(I)

worin

$R_1$ und $R_2$ unabhängig voneinander für Wasserstoff, Halogen, Methoxy oder Methylthio stehen;

$R_3$ Wasserstoff oder $C_1$–$C_4$-Haloalkyl bedeutet;

Y für Hydroxy, Halogen oder die Gruppe –O–C(O)–$R_4$ steht; und

$R_4$ $C_1$–$C_4$-Alkyl, $C_1$–$C_3$-Haloalkyl, $C_1$–$C_2$-Alkoxycarbonyl oder die Gruppe –CH($R_5$)–X$R_6$ bedeutet; wobei

X für Sauerstoff oder Schwefel steht;

$R_5$ Wasserstoff oder Methyl bedeutet; und

$R_6$ für $C_1$–$C_4$-Alkyl, ($C_1$–$C_2$-Alkoxy)-$C_1$–$C_2$-alkyl, $C_3$–$C_4$-Alkenyl oder Phenyl steht.

2. Verbindungen der Formel I gemäß Anspruch 1, dadurch gekennzeichnet, daß $R_1$ für H, 2-Cl, 2-Methoxy oder 2-Methylthio steht; $R_2$ H oder 3-Cl, bedeutet; $R_3$ für Wasserstoff oder $C_1$–$C_4$-Haloalkyl steht; und Y Hydroxy, Chlor oder die Gruppe –O–C(O)–$R_4$ bedeutet; wobei $R_4$ für $C_1$–$C_4$-Alkyl, $C_1$–$C_3$-Haloalkyl, $C_1$–$C_2$-Alkoxycarbonyl oder die Gruppe –$CH_2$–O$R_6$ bedeutet; wobei $R_6$ für $C_1$–$C_4$-Alkyl, ($C_1$–$C_2$-Alkoxy)-$C_1$–$C_2$-alkyl, $C_3$–$C_4$-Alkenyl oder Phenyl steht.

3. Verbindungen der Formel I gemäß Anspruch 2, dadurch gekennzeichnet, daß $R_1$ für H, 2-Cl, 2-Methoxy oder 2-Methylthio steht; $R_2$ H oder 3-Cl bedeutet; $R_3$ für Wasserstoff oder $CCl_3$ steht und Y Hydroxy, Chlor oder die Gruppe –O–C(O)–$R_4$ bedeutet; wobei $R_4$ für $C_1$–$C_4$-Alkyl, $C_1$–$C_2$-Haloalkyl, Methoxycarbonyl oder die Gruppe –$CH_2$O$R_6$ bedeutet; wobei $R_6$ für $C_1$–$C_4$-Alkyl, Methoxymethyl, Ethoxymethyl, Methoxyethyl, Allyl oder Phenyl steht.

4. Eine Verbindung der Formel I nach Anspruch 1, ausgewählt aus der Reihe:
N-(Hydroxymethyl)-3-(3-chlorphenyl)-4-cyanopyrrol,
N-(1-Hydroxy-2,2,2-trichlorethyl)-3-(3-methoxyphenyl)-4-cyanopyrrol,
N-(Chlormethyl)-3-(3-methoxyphenyl)-4-cyanopyrrol,
N-(1,2,2,2-Tetrachlorethyl)-3-(3-methoxyphenyl)-4-cyanopyrrol,
N-(1,2,2,2-Tetrachlorethyl)-3-(3-bromphenyl)-4-cyanopyrrol.

5. Eine Verbindung der Formel I nach Anspruch 1, ausgewählt aus der Reihe:
N-(1-Acetyloxy-2,2,2-trichlorethyl)-3-(2,3-dichlorphenyl)-4-cyanopyrrol,
N-(1-Hydroxy-2,2,2-trichlorethyl)-3-(2,3-dichlorphenyl)-4-cyanopyrrol,
N-(1-Methoxyacetyloxy-2,2,2-trichlorethyl)-3-(2,3-dichlorphenyl)-4-cyanopyrrol,
N-(Methoxyacetyloxymethyl)-3-(2,3-dichlorphenyl)-4-cyanopyrrol,
N-[1-(n-Butylcarbonyloxy)-2,2,2-trichlorethyl]-3-(2,3-dichlorphenyl)-4-cyanoprrol,
N-[1-(n-Propoxyacetyloxy)-2,2,2-trichlorethyl]-3-(2,3-dichlorphenyl)-4-cyanopyrrol,
N-[1-(n-Butoxyacetyloxy)-2,2,2-trichlorethyl]-3-(2,3-dichlorphenyl)-4-cyanopyrrol,
N-[1-(n-Butoxyacetyloxy)ethyl]-3-(2,3-dichlorphenyl)-4-cyanopyrrol,
N-[1-(Propargyloxyacetyloxy)-2,2,2-trichlorethyl]-3-(2,3-dichlorphenyl)-4-cyanopyrrol.

6. Eine Verbindung der Formel I nach Anspruch 1, ausgewählt aus der Reihe:
N-(1-Hydroxy-2,2,2-trichlorethyl)-3-(2-methylthiophenyl)-4-cyanopyrrol,
N-(1-Hydroxy-2,2,2-trichlorethyl)-3-(2-methoxyphenyl)-4-cyanopyrrol,
N-(1-Hydroxy-2,2,2-trichlorethyl)-3-(2-chlorphenyl)-4-cyanopyrrol,
N-[1-(Isopropoxyacetyloxy)-2,2,2-trichlorethyl]-3-(2-chlorphenyl)-4-cyanopyrrol,
N-[1-(Methoxyacetyloxy)-2,2,2-trichlorethyl]-3-(2-methylthiophenyl)-4-cyanopyrrol,
N-(Methoxyacetyl)-3-(2-chlorphenyl)-4-cyanopyrrol.

7. Verfahren zur Herstellung der in Anspruch 1 definierten Verbindungen der Formel I, dadurch gekennzeichnet, daß man in An- oder Abwesenheit eines Lösungsmittels bei Temperaturen zwischen 0° und 200 °C ein 3-Phenyl-4-cyanopyrrolderivat der Formel II

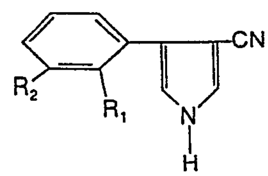

(II)

durch Reaktion mit einem Aldehyd der Formel III

$$R_3\text{–CHO} \qquad \text{(III)}$$

in An- oder Abwesenheit eines sauren oder basischen Katalysators in ein Hydroxyderivat der Formel Ia

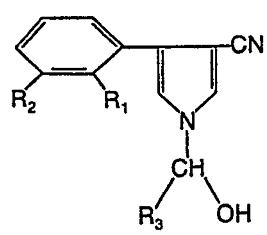

(Ia)

überführt und letzteres, sofern gewünscht, durch Austausch der freien OH-Gruppe gegen einen anderen Rest Y in ein anderes Derivat der Formel I umwandelt, wobei die Substituenten in den Formeln Ia, II und III sowie Y die unter Formel I angegebenen Bedeutungen haben.

8. Verfahren nach Anspruch 7, dadurch gekennzeichnet, daß man eine Verbindung der Formel Ia entweder in An- oder Abwesenheit eines üblichen

inerten Lösungsmittels mit einer Säure der Formel IV

$R_4$–COOH (IV)

oder einem ihrer reaktionsfähigen Säurederivate bei Temperaturen zwischen 0 und 150 °C direkt in ein Acyloxy-Produkt der Formel Ib

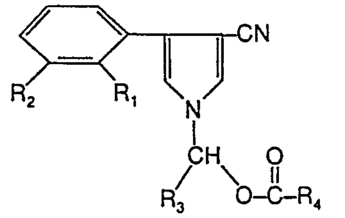

(Ib)

überführt oder dadurch, daß man die freie OH-Gruppe in einer Verbindung der Formel Ia in an sich bekannter Art durch ein Halogenatom austauscht und das entstandene halogenierte Produkt in An- oder Abwesenheit eines reaktionsinerten Lösungsmittels bei Temperaturen zwischen 0° und 150 °C und in An- oder Abwesenheit eines Reaktionskatalysators mit einem Salz der Formel V

$R_4$–$COO^-M^+$ (V)

in eine Verbindung der Formel Ib überführt, wobei die Substituenten in den Formeln Ib, IV und V wie unter Formel I definiert sind und $M^+$ für ein Metallkation steht.

9. Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, daß es als mindestens eine aktive Komponente eine Verbindung der Formel I enthält.

10. Mittel gemäß Anspruch 9, dadurch gekennzeichnet, daß es als aktive Komponente mindestens eine der in den Ansprüchen 1 bis 4 definierten Verbindungen enthält.

11. Mittel nach Anspruch 10, dadurch gekennzeichnet, daß es 0,1 bis 99% eines Wirkstoffs der Formel I, 99,9 bis 1% eines festen oder flüssigen Zusatzstoffes und 0 bis 25% eines Tensides enthält.

12. Mittel nach Anspruch 11, dadurch gekennzeichnet, daß es 0,1 bis 95% eines Wirkstoffes der Formel I 99,8 bis 5% eines festen oder flüssigen Zusatzstoffes und 0,1 bis 25% eines Tensides enthält.

13. Verfahren zur Bekämpfung oder Verhütung eines Befalls von Kulturpflanzen durch phytopathogene Mikroorganismen, dadurch gekennzeichnet, daß man eine Verbindung der Formel I auf die Pflanze, Pflanzenteile oder deren Standort appliziert.

14. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer Verbindung der Formel I.

## Claims

1. A compound of the formula I

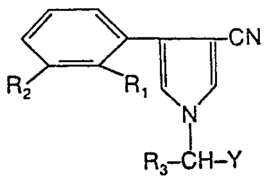

(I)

wherein

$R_1$ and $R_2$, each independently of the other, are hydrogen, halogen, methoxy or methylthio,

$R_3$ is hydrogen or $C_1$–$C_4$haloalkyl,

Y is hydroxy, halogen or the –O–C(O)–$R_4$ group; and

$R_4$ is $C_1$–$C_6$alkyl, $C_1$–$C_6$haloalkyl, 2-tetrahydrofuryl, 2-tetrahydropyranyl, $C_1$–$C_2$alkoxycarbonyl or the –CH($R_5$)–X$R_6$ group, wherein

X is oxygen or sulfur,

$R_5$ is hydrogen or methyl, and

$R_6$ is $C_1$–$C_6$alkyl, ($C_1$–$C_3$alkoxy)-$C_1$–$C_3$alkyl, $C_3$–$C_6$alkenyl, $C_3$–$C_6$alkynyl, phenyl or phenyl which is substituted by fluorine, chlorine, bromine, methyl, methoxy and/or $C_1$–$C_3$alkoxycarbonyl.

2. A compound of the formula I according to claim 1, wherein $R_1$ is hydrogen, 2-Cl, 2-methoxy or 2-methylthio; $R_2$ is hydrogen or 3-Cl; $R_3$ is hydrogen or $C_1$–$C_4$haloalkyl; and Y is OH, chlorine or the –O–C(O)–$R_4$ group, wherein $R_4$ is $C_1$–$C_4$alkyl, $C_1$–$C_3$haloalkyl, 2-tetrahydrofuryl, 2-tetrahydropyranyl, $C_1$–$C_2$alkoxycarbonyl or the –$CH_2$–$OR_6$ group, wherein $R_6$ is $C_1$–$C_4$alkyl, ($C_1$–$C_3$alkoxy)-$C_1$-$C_3$alkyl, $C_3$–$C_6$alkenyl or $C_3$–$C_6$-alkynyl, phenyl or phenyl which is substituted by fluorine, chlorine, bromine, methyl, methoxy, ethoxycarbonyl and/or methoxycarbonyl.

3. A compound of the formula I according to claim 2, wherein $R_1$ is hydrogen, 2-Cl, 2-methoxy or 2-methylthio; $R_2$ is hydrogen or 3-Cl; $R_3$ is hydrogen or $CCl_3$; and Y is OH, chlorine or the –O–C(O)–$R_4$ group, wherein $R_4$ is $C_1$–$C_4$alkyl, $C_1$–$C_2$haloalkyl, 2-tetrahydrofuryl, 2-tetrahydropyranyl, methoxycarbonyl or the –$CH_2OR_6$ group, wherein $R_6$ is $C_1$–$C_4$alkyl, methoxymethyl, ethoxymethyl, methoxyethyl, allyl, propargyl, phenyl or phenyl which is substituted by fluorine, chlorine, bromine, methyl, methoxy and/or methoxycarbonyl.

4. A compound of the formula I according to claim 1, selected from the group consisting of:

N-(hydroxymethyl)-3-(3-chlorophenyl)-4-cyanopyrrole,

N-(1-hydroxy-2,2,2-trichloroethyl)-3-(2-methylthiophenyl)-4-cyanopyrrole,

N-(chloromethyl)-3-(3-methoxyphenyl)-4-cyanopyrrole,

N-(1,2,2,2-tetrachloroethyl)-3-(3-methoxyphenyl)-4-cyanopyrrole,

N-(1,2,2,2-tetrachloroethyl)-3-(3-bromophenyl)-4-cyanopyrrole.

5. A compound of the formula I according to claim 1, selected from the group consisting of:

N-(1-acetoxy-2,2,2-trichloroethyl)-3-(2,3-dichlorophenyl)-4-cyanopyrrole,
N-(1-hydroxy-2,2,2-trichloroethyl)-3-(2,3-dichlorophenyl)-4-cyanopyrrole,
N-(1-methoxyacetoxy-2,2,2-trichloroethyl)-3-(2,3-dichlorophenyl)-4-cyanopyrrole,
N-(methoxyacetoxymethyl)-3-(2,3-dichlorophenyl)-4-cyanopyrrole,
N-[1-(n-butylcarbonyloxy)-2,2,2-trichloroethyl]-3-(2,3-dichlorophenyl)-4-cyanopyrrole,
N-[1-(n-propoxyacetoxy)-2,2,2-trichloroethyl]-3-(2,3-dichlorophenyl)-4-cyanopyrrole,
N-[1-(n-butoxyacetoxy)-2,2,2-trichloroethyl]-3-(2,3-dichlorophenyl)-4-cyanopyrrole,
N-[1-(n-butoxyacetoxy)ethyl]-3-(2,3-dichlorophenyl)-4-cyanopyrrole,
N-[1-(propargyloxyacetoxy)-2,2,2-trichloroethyl]-3-(2,3-dichlorophenyl)-4-cyanopyrrole.

6. A compound of the formula I according to claim 1, selected from the group consisting of:
N-(1-hydroxy-2,2,2-trichloroethyl)-3-(2-methylthiophenyl)-4-cyanopyrrole,
N-(1-hydroxy-2,2,2-trichloroethyl)-3-(2-methoxyphenyl)-4-cyanopyrrole,
N-(1-hydroxy-2,2,2-trichloroethyl)-3-(2-chlorophenyl)-4-cyanopyrrole,
N-[1-(isopropoxyacetoxy)-2,2,2-trichloroethyl]-3-(2-chlorophenyl)-4-cyanopyrrole,
N-[1-(methoxyacetoxy)-2,2,2-trichloroethyl]-3-(2-methylthiophenyl)-4-cyanopyrrole,
N-(methoxyacetyl)-3-(2-chlorophenyl)-4-cyanopyrrole.

7. A process for the preparation of a compound of the formula I as defined in claim 1, which comprises converting a 3-phenyl-4-cyanopyrrole derivative of the formula II

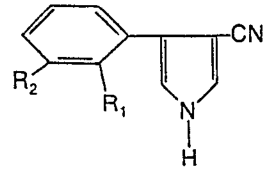 (II)

in the presence or absence of a solvent and in the temperature range from 0° to +200 °C, by reaction with an aldehyde of the formula III

$$R_3\text{–CHO} \qquad (III)$$

in the presence or absence of an acid or basic catalyst, into a hydroxy derivative of the formula Ia

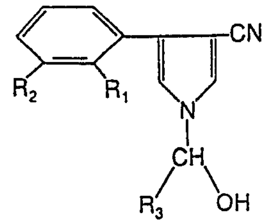 (Ia)

and, if desired, converting said derivative into another derivative of the formula I by replacing the free OH group by another radical Y, in which formulae Ia, II and III the substituents, as well as Y, are as defined for formula I.

8. A process according to claim 7, which comprises converting a compound of the formula Ia, either in the presence or absence of a customary inert solvent, with an acid of the formula IV

$$R_4\text{–COOH} \qquad (IV)$$

or with a reactive acid derivative thereof, in the temperature range from 0° to +150 °C, direct into an acyloxy compound of the formula Ib

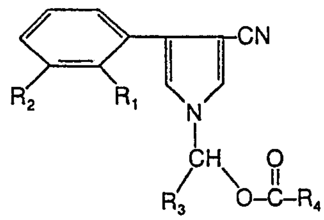 (Ib)

or replacing the free OH group in a compound of the formula Ia, in a manner known per se, by a halogen atom, and converting the halogenated compound so obtained, in the presence or absence of an inert solvent, in the temperature range from 0° to +150 °C, and in the presence or absence of a reaction catalyst, into a compound of the formula Ib with a salt of the formula V

$$R_4\text{–COO}^{\ominus}\ M^{\oplus} \qquad (V)$$

in which formulae Ib, IV and V the substituents are as defined for formula I and $M^{\oplus}$is a metal cation.

9. A microbicidal composition for controlling phytopathogenic microorganisms or for protecting living plants from attack by such microorganisms and/or for preserving perishable storable goods of vegetable or animal origin, which composition contains as at least one active component a compound as defined in claim 1.

10. A composition according to claim 9, which contains as at least one active component a compound as defined in any one of claims 1 to 4.

11. A composition according to claim 10, which comprises 0.1 to 99% of a compound of formula I, 99.9 to 1% of a solid or liquid adjuvant and 0 to 25% of a surfactant.

12. A composition according to claim 11, which comprises 0.1 to 95% of a compound of formula I, 99.8 to 5% of a solid or liquid adjuvant and 0.1 to 25% of a surfactant.

13. A method of controlling phytopathogenic microorganisms or of protecting cultivated plants from attack by said microorganisms, which method comprises applying to said plants, to part of plants or to the locus thereof a compound of the formula I.

14. A method of preserving or protecting storable goods of plant and/or animal origin from harmful microorganisms by treating said goods with at least a microbicidally effective amount of a compound of formula I.

**Revendications**

1. Composés de formule I

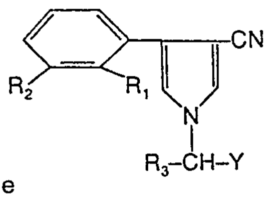

(I)

dans laquelle

$R_1$ et $R_2$ représentent chacun, indépendamment l'un de l'autre, l'hydrogène, un halogène, un groupe méthoxy ou méthylthio;

$R_3$ représente l'hydrogène ou un groupe halogénoalkyle en $C_1$–$C_4$;

Y représente un groupe hydroxy, un halogène ou le groupe $-O-C(O)-R_4$, et

$R_4$ représente un groupe alkyle en $C_1$–$C_6$, halogénoalkyle en $C_1$–$C_6$, 2-tétrahydrofuryle, 2-tétrahydropyrannyle, (alcoxy en $C_1$–$C_2$)-carbonyle ou le groupe $-CH(R_5)-XR_6$ dans lequel

X représente l'oxygène ou le soufre;

$R_5$ représente l'hydrogène ou un groupe méthyle; et

$R_6$ représente un goupe alkyle en $C_1$–$C_6$, (alcoxy en $C_1$–$C_3$)-alkyle en $C_1$–$C_3$, alcényle en $C_3$–$C_6$, alcynyle en $C_3$–$C_6$ ou phényle non substitué ou substitué par le fluor, le chlore, le brome, des groupes méthyle, méthoxy et/ou (alcoxy en $C_1$–$C_3$)-carbonyle.

2. Composés de formule I selon la revendication 1, caractérisés en ce que $R_1$ représente H, un atome de chlore en position 2, un groupe méthoxy en position 2 ou un groupe méthylthio en position 2; $R_2$ représente H ou un atome de chlore en position 3; $R_3$ représente l'hydrogène ou un groupe halogénoalkyle en $C_1$–$C_4$; et Y représente un groupe hydroxy, le chlore ou le groupe $-O-C(O)-R_4$ dans lequel $R_4$ représente un groupe alkyle en C 1-C 4, halogénoalkyle en C 1-C 3, 2–tétra–hydrofuryle, 2-tétrahydropyrannyle, (alcoxy en $C_1$–$C_2$)-carbonyle ou le groupe $-CH_2-OR_6$ dans lequel $R_6$ représente un groupe alkyle en $C_1$–$C_4$, (alcoxy en $C_1$–$C_3$)-alkyle en $C_1$–$C_3$, alcényle en $C_3$–$C_6$, alcynyle en $C_3$–$C_6$ ou phényle non substitué ou substitué par le fluor, le chlore, le brome, des groupes méthyle, méthoxy, éthoxycarbonyle et/ou méthoxycarbonyle.

3. Composés de formule I selon la revendication 2, caractérisés en ce que $R_1$ représente H, un atome de chlore en position 2, un groupe méthoxy en position 2 ou un groupe méthylthio en position 2; $R_2$ représente H ou un atome de chlore en position 3; $R_3$ représente l'hydrogène ou le groupe $CCl_3$ et Y représente un groupe hydroxy, le chlore ou le groupe $-O-C(O)-R_4$ dans lequel $R_4$ représente un groupe alkyle en $C_1$–$C_4$, halogénoalkyle en $C_1$–$C_2$, 2-tétrahydrofuryle, 2-tétrahydropyrannyle, méthoxycarbonyle ou le groupe $-CH_2OR_6$ dans lequel $R_6$ représente un groupe alkyle en $C_1$–$C_4$, méthoxyméthyle, éthoxyméthyle, méthoxyéthyle, allyle, propargyle, phényle ou phényle substitué par le fluor, le chlore, le brome, des groupes méthyle, méthoxy et/ou méthoxycarbonyle.

4. Un composé de formule I selon la revendication 1, choisi dans le groupe suivant:

N-(hydroxyméthyl)-3-(3-chlorophényl)-4-cyanopyrrole,
N-(1-hydroxy-2,2,2-trichloréthyl)-3-(3-méthoxyphényl)-4-cyanopyrrole,
N-(chlorométhyl)-3-(3-méthoxyphényl)-4-cyanopyrrole,
N-(1,2,2,2-tétrachloréthyl)-3-(3-méthoxyphényl)-4-cyanopyrrole,
N-(1,2,2,2-tétrachloréthyl)-3-(3-bromophényl)-4-cyanopyrrole.

5. Un composé de formule I selon la revendication 1, choisi dans le groupe suivant:

N-(1-acétyloxy-2,2,2-trichloréthyl)-3-(2,3-dichlorophényl)-4-cyanopyrrole,
N-(1-hydroxy-2,2,2-trichloréthyl)-3-(2,3-dichlorophényl)-4-cyanopyrrole,
N-(1-méthoxyacétyloxy-2,2,2-trichloréthyl)-3-(2,3-dichlorophényl)-4-cyanopyrrole,
N-(méthoxyacétyloxyméthyl)-3-(2,3-dichlorophényl)-4-cyanopyrrole,
N-[1-(n-butylcarbonyloxy)-2,2,2-trichloréthyl]-3-(2,3-dichlorophényl)-4-cyanopyrrole,
N-[1-(n-propoxyacétyloxy)-2,2,2-trichloréthyl]-3-(2,3-dichlorophényl)-4-cyanopyrrole,
N-[1-(n-butoxyacétyloxy)-2,2,2-trichloréthyl]-3-(2,3-dichlorophényl)-4-cyanopyrrole,
N-[1-(n-butoxyacétyloxy)-éthyl]-3-(2,3-dichlorophényl)-4-cyanopyrrole,
N-[1-(propargyloxyacétyloxy)-2,2,2-trichloréthyl]-3-(2,3-dichlorophényl)-4-cyanopyrrole.

6. Un composé de formule I selon la revendication 1, choisi dans le groupe suivant:

N-(1-hydroxy-2,2,2-trichloréthyl)-3-(2-méthylthiophényl)-4-cyanopyrrole,
N-(1-hydroxy-2,2,2-trichloréthyl)-3-(2-méthoxyphényl)-4-cyanopyrrole,
N-(1-hydroxy-2,2,2-trichloréthyl)-3-(2-chlorophényl)-4-cyanopyrrole,
N-[1-(isopropoxyacétyloxy)-2,2,2-trichloréthyl]-3-(2-chlorophényl)-4-cyanopyrrole,
N-[1-(méthoxyacétyloxy)-2,2,2-trichloréthyl]-3-(2-méthylthiophényl)-4-cyanopyrrole,
N-(méthoxyacétyl)-3-(2-chlorophényl)-4-cyanopyrrole.

7. Procédé de préparation des composés de formule I définis dans la revendication 1, caractérisé en ce que l'on convertit en présence ou en l'absence d'un solvant, à des températures de 0 à 200 °C, un dérivé du 3-phényl-4-cyanopyrrole de formule II

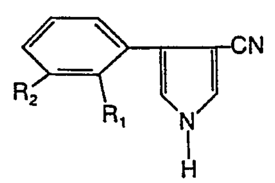

(II)

par réaction avec un aldéhyde de formule III

$$R_3-CHO \qquad (III)$$

en présence ou en l'absence d'un catalyseur acide ou basique, en un dérivé hydroxylé de formule Ia

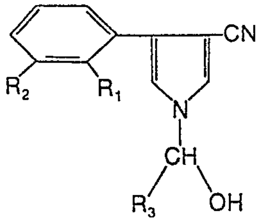

(Ia)

qu'on convertit lui-même si on le désire, par échange du groupe OH libre contre un autre substituant Y, en un autre dérivé de formule I, les symboles des formules Ia, II et III ainsi que Y ayant les significations indiquées en référence à la formule I.

8. Procédé selon la revendication 7, caractérisé en ce que l'on convertit directement un composé de formule Ia en présence ou en l'absence d'un solvant usuel inerte, à l'aide d'un acide de formule IV

$R_4$–COOH (IV)

ou d'un dérivé d'un tel acide, à des températures de 0 à 150 °C, en un composé acyloxylé de formule Ib

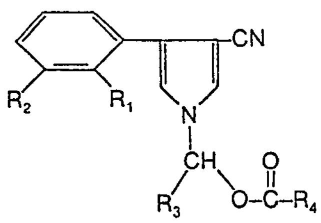

(Ib)

ou bien dans un composé de formule Ia, on échange le groupe OH libre, de manière connue en soi, contre un atome d'halogène et on convertit le produit halogéné obtenu en présence ou en l'absence d'un solvant inerte dans la réaction, à des températures de 0 à 150 °C et en présence ou en l'absence d'un catalyseur de réaction, à l'aide d'un sel de formule V

$R_4$–$COO^-M^+$ (V)

en un composé de formule Ib, les symboles des formules Ib, IV et V ayant les significations indiquées en référence à la formule I et $M^+$ représentant un cation métallique.

9. Produit microbicide pour combattre ou prévenir une infestation de végétaux vivantes et/ou pour conserver des denrées périssables d'origine végétale ou animale, caractérisé en ce qu'il contient au moins un composant actif consistant en un composé de formule I.

10. Produit selon la revendication 9, caractérisé en ce qu'il contient en tant que composant actif au moins l'un des composés définis dans les revendications 1 à 4.

11. Produit selon la revendication 10, caractérisé en ce qu'il contient de 0,1 à 99% d'une substance active de formule I, de 99,9 à 1% d'un additif solide ou liquide et de 0 à 25% d'un agent tensioactif.

12. Produit selon la revendication 11, caractérisé en ce qu'il contient de 0,1 à 95% d'une substance active de formule I, de 99,8 à 5% d'un additif solide ou liquide et de 0,1 à 25% d'un agent tensioactif.

13. Procédé pour combattre ou prévenir une infestation de végétaux cultivés par des microorganismes phytopathogènes, caractérisé en ce que l'on applique à la plante, à des parties de la plante ou à son habitat, un composé de formule I.

14. Procédé pour conserver ou protéger des réserves ou des stocks de matières d'origine végétale et/ou animale contre les microorganismes nuisibles par traitement de la matière par une quantité microbicide efficace d'un composé de formule I.